# EUROPEAN PATENT SPECIFICATION

(11) **EP 0 846 173 B1**
(45) Date of publication and mention of the grant of the patent: **19.10.2005**
(21) Application number: 96929657.3
(22) Date of filing: 23.08.1996
(51) Int. Cl.: C12N 15/24, C07K 14/54, A61K 38/20, A61K 38/04, A61K 39/395, C12N 15/11, C12N 1/21, C12N 15/09, C07K 16/24, C12N 5/12, C12N 5/10, G01N 33/53

(54) **USE OF AN ANTI-INTERLEUKIN-9 ANTIBODY FOR THE PREPARATION OF A MEDICAMENT FOR THE TREATMENT OF ASTHMA**
VERWENDUNG EINES ANTI-INTERLEUKIN-9 ANTIKÖRPERS ZUR HERSTELLUNG EINES MEDIKAMENTES ZUR BEHANDLUNG VON ASTHMA
UTILISATION D'UN ANTICORPS ANTI-INTERLEUKIN-9 POUR LA PREPARATION D'UN MEDICAMENT POUR LE TRAITEMENT DE L'ASTHME

(30) Priority: 24.08.1995 US 2765 P; 06.08.1996 US 23800 P
(43) Date of publication of application: 10.06.1998
(60) Divisional application: 05009688.2
(73) Proprietor: GENAERA CORPORATION, Plymouth Meeting, PA 19462 (US)
(72) Inventor: LEVITT, Roy, Clifford, Ambler, PA 19002 (US); Maloy, W. Lee, Lansdale, PA 19446 (US); KARI, Prasad, U, Lansdale, PA 19440 (US); NICOLAIDES, Nicolas, Boothwyn, Pennsylvania 19061 (US)
(74) Representative: Polz, Leo
(86) International application number: PCT/US1996/012757
(87) International publication number: WO 1997/008321

(56) References cited:
- EP-A- 0 361 284
- WO-A-92/05698
- J. IMMUNOLOGY, vol. 144, no. 11, 1 June 1990, pages 4235-4241, XP002024248 RENAULD J.-C. ET AL.: " Human P40/IL-9: Expression in activated CD4+ T-cells, genomic organization with the mouse gene."
- GENOMICS, vol. 23, 1994, pages 464-470, XP000616442 MEYERS D.A. ET AL. : "Evidence for a locus relating total serum IgE levels mapping to chromosome 5." cited in the application
- PROC.NATL.ACAD.SCI., vol. 89, June 1992, pages 5690-5694, XP002024249 RENAULD J.-C. ET AL.: " Expression cloning of the murine and human interleukin 9 receptor cDNAs." cited in the application
- FASEB J., vol. 2, 1988, pages 2605-2608, XP002024250 LEVITT R.C. AND MITZNER W.: "Expression of airway hyperreactivity to acetylcholine as a simple autosomal recessive trait in mice." cited in the application
- J. BIOL. CHEM., vol. 269, no. 43, 1994, pages 26614-26617, XP000616424 YIN T. ET AL.: "JAK1 kinase forms complexes with Interleukin-4 receptor and 4PS/Insulin receptor substrate-1-like protein and is activated by interleukin-9 in T-lymphocytes." cited in the application
- AM. J. RESPIR. CRIT. CARE MED., vol. 151, June 1995, pages 2065-2069, XP000616450 MOSSMAN B.T. ET AL. : "Advances in molecular genetics, transgenic models, and gene therapyfor the study of pulmonary diseases."
- INT. ARCH. ALLERGY IMMUNOL, vol. 107, July 1995, pages 29-33, XP000616471 HOLGATE S.T. ET AL.: "Genetic and environmental influences on airway inflammation in asthma."
- AMERICAN JOURNAL OF HUMAN GENETICS, vol. 55, no. 3s, September 1994, page 1120 XP000616459 LEVITT R.C. ET AL.: "A locus regulating bronchial hyperresponsiveness maps to chromosome 5q."

## Description

### CROSS-REFERENCE TO RELATED APPLICATION

This application is related to U.S. Provisional Application Serial No. 60/002,765 which was filed August 24, 1995.

### FIELD OF THE INVENTION

This invention elates to the use of an antibody for the preparation of a medicament for the treatment of asthma, based upon the relationship between IL-9 and its receptor.

### BACKGROUND OF THE INVENTION

Inflammation is a complex process in which the body's defense system combats foreign entities. While the battle against foreign entities may be necessary for the body's survival, some defense systems improperly respond to foreign entities, even innocuous ones, as dangerous and thereby damage surrounding tissue in the ensuing battle.

Atopic allergy is an ecogenetic disorder, where genetic background dictates the response to environmental stimuli. The disorder is generally characterized by an increased ability of lymphocytes to produce IgE antibodies in response to ubiquitous antigens. Activation of the immune system by these antigens leads to allergic inflammation and may occur after ingestion, penetration through the skin, or after inhalation. When this immune activation occurs and pulmonary inflammation ensues this disorder is broadly characterized as asthma. Certain cells are critical to this inflammatory reaction and they include T cells and antigen presenting cells, B cells that produce IgE, and mast cells/basophils and eosinophils that bind IgE. These inflammatory cells accumulate at the site of allergic inflammation and the toxic products they release contribute to the tissue destruction related to the disorder.

While asthma is generally defined as an inflammatory disorder of the airways, clinical symptoms arise from intermittent air flow obstruction. It is a chronic disabling disorder that appears to be increasing in prevalence and severity¹. It is estimated that 30-40% of the population suffer with atopic allergy, and 15% of children and 5% of adults in the population suffer from asthma.¹ Thus, an enormous burden is placed on our health care resources.

The mechanism of susceptibility to atopy and asthma remains unknown. Interestingly, while most individuals experience similar environmental exposures, only certain individuals develop atopic allergy and asthma. This hypersensitivity to environmental allergens known as "atopy" is often indicated by elevated serum IgE levels or abnormally great skin test response to allergens in atopic individuals as compared to nonatopics.¹⁰ Strong evidence for a close relationship between atopic allergy and asthma is derived from the fact that most asthmatics have clinical and serologic evidence of atopy.⁴⁻⁹ In particular, younger asthmatics have a high incidence of atopy.¹⁰ In addition, immunologic factors associated with an increase in serum total IgE levels are very closely related to impaired pulmonary function.³

Both the diagnosis and treatment of these disorders are problematic. The assessment of inflamed lung tissue is often difficult, and frequently the source of the inflammation cannot be determined. Without knowledge of the source of the airway inflammation and protection from the inciting foreign environmental agent or agents, the inflammatory process cannot be interrupted. It is now generally accepted that failure to control the pulmonary inflammation leads to significant loss of lung function over time.

Current treatments suffer their own set of disadvantages. The main therapeutic agents, β agonists, reduce the symptoms, i.e., transiently improve pulmonary functions, but do not affect the underlying inflammation so that lung tissue remains in jeopardy. In addition, constant use of β agonists results in desensitization which reduces their efficacy and safety.² The agents that can diminish the underlying inflammation, the anti-inflammatory steroids, have their own known list of disadvantages that range from immunosuppression to bone loss.²

Because of the problems associated with conventional therapies, alternative treatment strategies have been evaluated.⁶⁵⁻⁶⁶ Glycophorin A,⁶⁴ cyclosporin,⁶⁵ and a nonapeptide fragment of IL-2,⁶³ all inhibit interleukin-2 dependent T lymphocyte proliferation and therefore, IL-9 production,⁵¹ however, they are known to have many other effects.³ For example, cyclosporin is used as a immunosuppressant after organ transplantation. While these agents may represent alternatives to steroids in the treatment of asthmatics,⁶³⁻⁶⁶ they inhibit interleukin-2 dependent T lymphocyte proliferation and potentially critical immune functions associated with homeostasis. What is needed in the art is the identification of a pathway critical to the development of asthma that explains the episodic nature of the disorder and the close association with allergy that is downstream of these critical immune functions. Nature demonstrated that this pathway is the appropriate target for therapy since biologic variability normally exists at this pathway and these individuals are otherwise generally not immunocompromised or ill except for their symptoms of atopy.

Because of the difficulties related to the diagnosis and treatment of asthma, the complex pathophysiology of this disorder is under intensive study. Although this disorder is heterogeneous and may be difficult to define because it can take many forms, certain features are found in common among asthmatics. Examples of such traits include elevated serum IgE levels, abnormal skin test response to allergen challenge, bronchial hyperresponsiveness [BHR], bronchodilator reversibility, and airflow obstruction.³⁻¹⁰ These expressions of these asthma related phenotypes may be studied as quantitative or qualitative measures.

Elevated IgE levels are also closely correlated with BHR, a heightened bronchoconstrictor response to a variety of stimuli.^{4,6,8,9} BHR is believed to reflect the presence of airway inflammation,^{6,8} and is considered a risk factor for asthma.¹¹⁻¹² BHR is accompanied by bronchial inflammation and an allergic diathesis in asthmatic individuals.¹³⁻²¹ Even in children with no symptoms of atopy and asthma, BHR is strongly associated with elevated IgE levels.¹⁹

A number of studies document a heritable component to atopy and asthma.^{4,10,21} However, family studies have been difficult to interpret since these disorders are significantly influenced by age and gender, as well as many environmental factors such as allergens, viral infections, and pollutants.²²⁻²⁴ Moreover, because there is no known biochemical defect associated with susceptibility to these disorders, the mutant genes and their abnormal gene products can only be recognized by the anomalous phenotypes they produce. Thus, an important first step in isolating and characterizing a heritable component is identifying the chromosomal locations of the genes.

Cookson et al. provided the first description of a genetic localization for inherited atopy.²⁵ These investigators described evidence for genetic linkage between atopy and a single marker on a specific chromosomal region designated 11q13.1. Later, they suggested evidence of maternal inheritance for atopy at this locus.²⁶ Although maternal inheritance [genetic imprinting] had been observed for atopy, it had never been explained previously. However, efforts to confirm this linkage have not been generally successful.²⁷⁻³³

Recently, the β subunit of the high-affinity IgE receptor was mapped to chromosome 11q, and a putative mutation associated with atopy has been described in this gene.^{32,33} However, because of the difficulties by others of replicating this linkage, the significance of this gene and polymorphism remains unclear. While additional studies will be required to confirm whether this putative mutation causes atopy in the general population, data collected so far suggests this polymorphism is unlikely to represent a frequent cause of atopy.

Because serum IgE levels are so closely associated with the onset and severity of allergy and asthma as clinical disorders, attention has focused on studies of the genetic regulation of serum total IgE levels. While past studies have provided evidence for Mendelian inheritance for serum total IgE levels,³⁴⁻³⁸ an indication of the existence of one regulatory gene, others have found evidence for polygenic inheritance of IgE, i.e., existence of several responsible genes.³⁹

Artisans have found several genes that may be important in the regulation of IgE and the development or progression of bronchial inflammation associated with asthma on chromosome 5q. They include genes encoding several interleukins, such as IL-3, IL-4, IL-5, IL-9, IL-13, granulocyte macrophage colony stimulating factor [GM CSF], a receptor for macrophage colony stimulating factor [CSF-1R], fibroblast growth factor acidic [FGFA], as well as others.⁴⁰ Recent evidence from family studies suggests genetic linkage between serum IgE levels and DNA markers in the region of these candidate genes on chromosome 5q.^{41,42} Together, these investigations suggest that one or more major genes in the vicinity of the interleukin complex on chromosome 5q regulates a significant amount of the observed biologic variability in serum IgE that is likely to be important in the development of atopy and asthma.

Linkage [sib-pair analyses] was also used previously to identify a genetic localization for BHR.⁷⁹ Because BHR was known to be associated with a major gene for atopy, chromosomal regions reported to be important in the regulation of serum IgE levels were examined.⁴² Candidate regions for atopy have been identified by linkage analyses. These studies identified the existence of a major gene for atopy on human chromosome 5q31-q33.⁴²

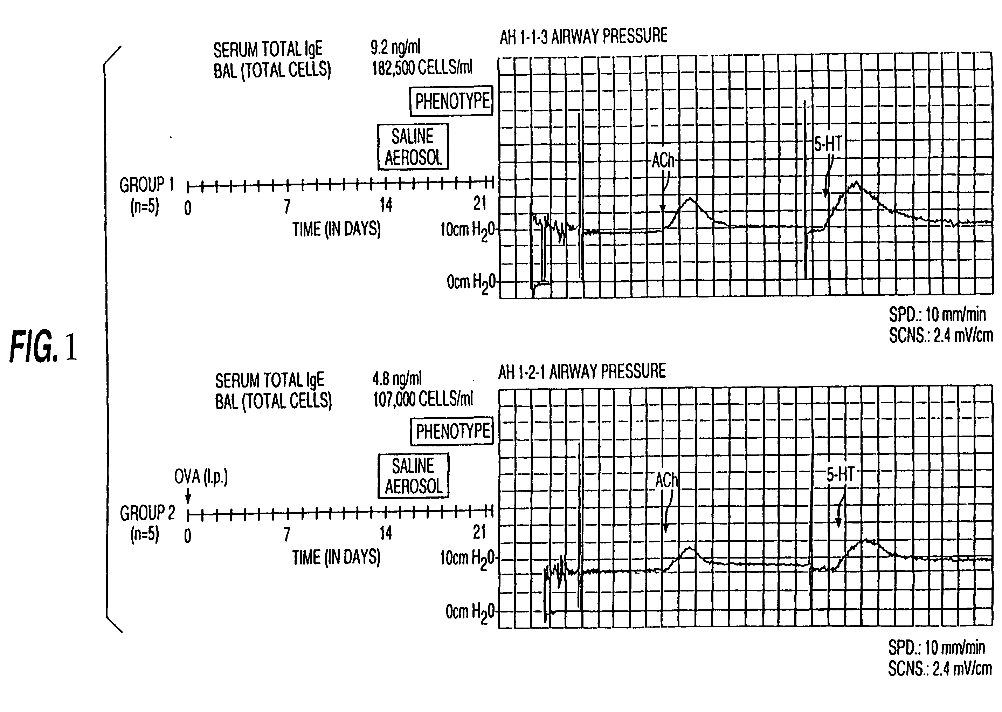

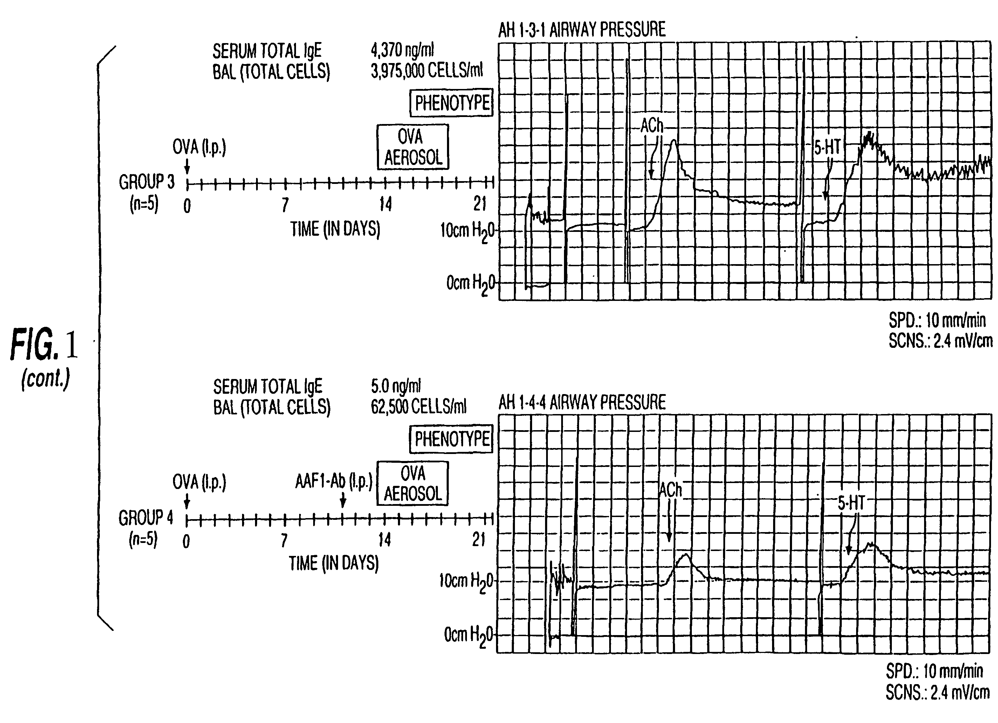

Therefore, to determine the chromosomal location of a gene[s] providing susceptibility to BHR, which would be coinherited with a major gene for atopy, experiments were carried out using linkage analyses between BHR and genetic markers on chromosome Sq.^{42,79,82} Individuals with BHR were identified by responsiveness to histamine. Markers useful for mapping asthma-related genes are shown in Figure 1.

Specifically, gene candidates for asthma, bronchial hyperresponsiveness, and atopy are shown [right] in their approximate location relative to the markers shown. The map includes the interleukin genes IL-4, IL-13, IL-5, and IL-3; CDC25, cell division cycle-25; CSF2, granulocyte-macrophage colony stimulating factor [GMCSF]; EGR1 early growth response gene-1; CD14, cell antigen 14; ADRB2, the β2-adrenergic receptor; GRL1, lymphocyte-specific glucocorticoid receptor; PDGFR, platelet-derived growth factor receptor. Bands 5q31-q33 extend approximately from IL-4 to D5S410. The distances reported are sex-averaged recombination fractions.

Affected sib-pair analyses demonstrated statistically significant evidence for linkage between BHR and D5S436, D5S658, and several other markers located nearby on chromosome 5q31-q33.⁷⁹ These data strongly supported the hypothesis that one or more closely spaced gene[s] on chromosome 5q31-q33 determine susceptibility to BHR, atopy, and asthma.^{79,80,81,83}

Recently linkage has also been demonstrated between the asthma phenotype and genetic markers on chromosome 5q31-q33.⁸³ This region of the human genome was evaluated for linkage with asthma because of the large number of genes representing reasonable positional candidates for providing genetic susceptibility for atopy and BHR.

Linkage was demonstrated using the methods described above.^{42,83} Specifically, 84 families were analyzed from the Netherlands with both sib-pair and LODs for markers from this same region of chromosome 5q previously shown to be linked to BHR and atopy.^{42,83} An algorithm was used to categorize obstructive airways disease in the asthmatic probands and their families. This classification scheme was based, as described previously, on the presence or absence of BHR to histamine, respiratory symptoms, significant smoking history [>5 pack years], atopy as defined by skin test response, airway obstruction [FEV1 % predicted < 95% CI] and reversibility to a bronchoilator [> 9% predicted].

Evidence was found for linkage between asthma and markers on chromosome 5q by affected sib pair analysis (N=10, P<0.05) and by maximum likelihood analysis with a dominant model for the asthma phenotype.⁸³

Asthma was linked to D5S658 with a maximal LOD of 3.64 at θ = 0.03, using a dominant model [class 1 affected, class 2-4 uncertain, class 5 unaffected] with a gene frequency of 0.015 [prevalence of 3%]. A maximal LOD of 2.71 at θ = 0.0 was observed for D5S470 which is approximately 5cM telomeric, or away from IL-9, relative to D5S436.⁸³

Subsequent to the original filing of this application, IL-9 or a gene nearby was suggested as likely to be important use atopy and asthma.⁴³ The IL-9 suggestion was based on a strong correlation in a randomly ascertained population between log serum total IgE levels and alleles of a genetic marker in the IL-9 gene.⁴³ This type of association with one or more specific alleles of a marker is termed "linkage disequilibrium", and generally suggests that a nearby gene determines the biologic variability under study.⁴⁴

The IL-9 gene has been mapped to the q31-q33 region of chromosome 5.⁴⁰ Only a single copy of the gene is found in the human genome.^{45,46} Structural similarity has been observed for the human and murine IL-9 genes.^{45,46} Each gene consists of five exons and four introns. extending across approximately four Kb of DNA. Expression of the gene appears to be restricted to activated T cells.^{45,46}

The functions of IL-9 now extend well beyond those originally recognized. While IL-9 serves as a T cell growth factor, this cytokine is also known to mediate the growth of erythroid progenitors, B cells, mast cells, and fetal thymocytes.^{45,46} IL-9 acts synergistically with IL-3 in causing mast cell activation and proliferation.⁴⁷ This cytokine also potentiates the IL-4 induced production of IgE, IgG, and IgM by normal human B lymphocytes.⁴⁸ IL-9 also potentiates the IL-4 induced release of IgE and IgG1 by murine B lymphocytes.⁴⁹ A critical role for IL-9 in the mucosal inflammatory response to parasitic infection has also been demonstrated.^{50,52}

In addition to IL-9, chromosome 5q bears numerous other gene candidates including IL-3, IRF1, EGR1, ITK, GRL1, ADRB2, CSF1R, FGFA, ITGA2,CD14, PDGFR, CDC25, CSF2, IL-4, IL-5, IL-12B, and IL-13. These may all be important in atopic allergy and as potential targets for therapeutic development. Moreover, the art lacks any knowledge regarding how the sequence of IL-9 or the function of IL-9 specifically correlates with atopic allergy, asthma, or bronchial hyperresponsiveness. Without such knowledge, artisans would not know how or whether to use IL-9 to either diagnose or treat these disorders.

The art does provide that IL-9 is a novel cytokine having an apparent molecular weight of approximately between 20 to 30 kD as determined by sodium dodecyl sulfate polyacrylamide gel electrophoresis under reducing conditions. It is produced as a 144 amino acid protein, that is processed to a 126 amino acid glycoprotein. Yang et al.⁸⁵ disclose that the DNA sequence encoding IL-9 comprises approximately 630 nucleotides, with approximately 450 nucleotides in the proper reading frame for the protein.

It is also known in the art that multiple protein isoforms may be generated from a single genetic locus by alternative splicing. Alternative splicing is an efficient mechanism by which multiple protein isoforms may be generated from a single genetic locus. Alternative splicing is used in terminally differentiated cells to reversibly modify protein expression without changing the genetic content of the cells. These protein isoforms are preferentially expressed in different tissues or during different states of cell differentiation or activation. Protein isoforms may have different functions and Alms and White have cloned and expressed a naturally occurring splice variant of IL-4, formed by the omission of exon 2, thus called IL-4δ2.⁸⁶ It was observed that IL-4δ2 inhibits T-cell proliferation induced by IL-4.

However, the art lacks any knowledge about IL-9 protein isoforms which are formed by deletions of exons 2 and 3 or the regulatory functions exhibited by these truncated proteins. Specifically, their role in regulating the biological activity, namely, the down-regulation of IL-9 expression or activity is unclear. Moreover, the formation of such isoforms by alternative splicing has not been previously observed or used to provide variants of IL-9 which function as agonists or antagonists of the native cytokine.

The art also lacks any knowledge about the role of the IL-9 receptor with asthma-related disorders. It is known that IL-9 binds to a specific receptor expressed on the surface of target cells.^{46,52,53} The receptor actually consists of two protein chains: one protein chain, known as the IL-9 receptor, binds specifically with IL-9 and the other protein chain is the chain, which is shared in common with the IL-2 receptor.⁴⁶ In addition, the human IL-9 receptor cDNA has been cloned.^{46,52,53} This cDNA encodes a 522 amino acid protein which exhibits significant homology to the murine IL-9 receptor. The extracellular region of the receptor is highly conserved, with 67% homology existing between the murine and human proteins. The cytoplasmic region of the receptor is less highly conserved. The human cytoplasmic domain is much larger than the corresponding region of the murine receptor.⁴⁶

The IL-9 receptor gene has also been characterized.⁵³ It is thought to exist as a single copy in the mouse genome and is composed of nine exons and eight introns.⁵³ The human genome contains at least four IL-9 receptor pseudogenes. The human IL-9 receptor gene has been mapped to the 320 kb subtelomeric region of the sex chromosomes X and Y.⁴⁶ Nonetheless, despite these studies, the art lacks any knowledge of a relation between the IL-9 receptor and atopic allergy, asthma, or bronchial hyperresponsiveness.

Thus, the art lacks any knowledge of how the IL-9 gene, its receptor, and their functions, are related to asthma. Therefore, there is a specific need in the art for genetic information on asthma and for elucidation of the role of IL-9 in the etiology of this disorder. There is also a need for elucidation of the role of the IL-9 receptor and the IL-9 receptor gene in this disorder. Furthermore, most significantly, based on this knowledge, there is a need for the identification of agents i.e. antibodies, which are capable of regulating the interaction between IL-9 and its receptor for treating this disorder.

### SUMMARY OF THE INVENTION

Applicant has satisfied the long felt need for a treatment of asthma by providing information demonstrating the role of IL-9 (also known as Asthma Associated Factor 1, or AAFI) in the pathogenesis of this disorder which information has led to compounds that are capable of regulating the activity of IL-9. Applicant has also demonstrated conserved linkage and synteny homologies between mice and humans for a gene that determines biologic variability in airway hyperresponsiveness. These relationships specifically identify IL-9 as a gene candidate. In addition, applicant has determined that IL-9 is critical to a number of antigen-induced responses in mice including bronchial hyperresponsiveness, eosinophilia and elevated cell counts in bronchial lavage, and elevated serum total IgE. These findings typify the allergic inflammation associated with asthma.

Polyclonal and monoclonal antibodies which block the binding of IL-9 to its receptor are claimed in this invention and are useful therapeutic agents in treating asthma.

The accompanying figures, which are incorporated in and constitute a part of this specification, illustrate several embodiments of the invention and, together with the description, serve to explain the principle of the invention.

### BRIEF DESCRIPTION OF THE DRAWINGS

**Figure 1.** Characterization of the role of IL-9 in the antigen response in vivo.
**Figure 2:** Histologic examination of lungs from control, ova challenged, and anti-IL-9 pretreated animals.
**Figure 3:** Inhibition of the antigen response in vivo by blocking antibodies to the murine IL-9 receptor.

### DETAILED DESCRIPTION OF THE INVENTION

Applicant has resolved the needs in the art by elucidating an IL-9 pathway and compositions that affect that pathway that may be used in the diagnosis, prevention or treatment of asthma. Asthma encompasses inflammatory disorders of the airways with reversible airflow obstruction.

Airway hyperresponsiveness is found in virtually all asthmatics and in some strains of inbred mice (DBA/2).⁸⁴ Airway hyperresponsiveness is a risk factor for the development of asthma in humans and is used in animal models of asthma as a physiologic measure to assess the efficacy of treatment for asthma. These data along with human⁷⁹ and murine genetic mapping results (see Examples 1 and 2) suggest a critical role for the murine IL-9 gene product in the airway response of the mouse. In particular, the hyperresponsive DBA/2(D2) mice differ from the C57BL/6(B6)hyporesponsive mice⁸⁴ in their expression of steady state levels of IL-9 (See Example 14, Figure 26). Furthermore, pretreatment with blocking antibodies to IL-9/IL-9 receptor can optionally provide complete protection from antigen induced airway hyperresponsiveness and inflammation in mice demonstrating a critical regulatory role for IL-9 in these immune responses. Thus, these data demonstrate that although different molecular changes produce biologic variability in airway responsiveness in humans and mice, these changes arise in the same gene(s) (IL-9/IL-9R) that regulate this pathway. Taken together, these observations confirm the critical role of IL-9 and the IL-9 receptor in airway hyperresponsiveness, asthma, and atopic allergy. Moreover, these data demonstrate that agents of the convention, which block the interaction of IL-9 with its receptor, protect against an antigen induced response such as those detailed above.

Further evidence defining the critical role of IL-9 in the pathogenesis of asthma derives directly from the applicants observation that IL-9 is critical to a number of antigen induced responses in mice. When the functions of IL-9 are down regulated by antibody pretreatment prior to aerosol challenge with antigen, the animals can be completely protected from the antigen induced responses. These responses include: bronchial hyperresponsiveness, eosinophilia and elevated cell counts in bronchial lavage, histologic changes in lung associated with inflammation, and elevated serum total IgE. Thus, the treatment of such responses, which characterize the allergic inflammation associated with asthma, by the down regulation of the functions of IL-9, are within the scope of this invention.

The skilled artisan will readily recognize that all molecules containing the requisite 3-dimensional structural conformation and which contain the residues essential or critical for receptor binding are within the scope of this invention.

The demonstration of an IL-9 sequence associated with an asthma-like phenotype, and one associated with the lack of an asthma-like phenotype, indicates that the lungs' inflammatory response to antigen is dependent on IL-9, and therefore, that down regulating the function of IL-9 should protect against the antigen induced response.

A receptor is a soluble or membrane bound component that recognizes and binds to molecules, and the IL-9 receptor (also known as Asthma Associated Factor 2 or AAF2) of the invention is the component that recognizes and binds to IL-9. The functions of the IL-9 receptor consist of binding an IL-9-like molecule and propagating its regulatory signal in specific cells.⁵⁷⁻⁶⁰ An interruption of that function will lead to a down regulation, i.e., reduction, of either the expression of IL-9 or of the functions controlled by IL-9. Accordingly, by virtue of this interaction between IL-9 and the IL-9 receptor, certain functions of rhe organism are modulated or controlled. For a general discussion of receptors, see Goodman and Gilman's The Pharmacologic Basis of Therapeutics (Seventh Edition, MacMillan Publishing Company, N.Y. USA, 1985).

The invention involves treatment of asthma.

In addition, this invention also provides compounds that prevent the synthesis or reduce the biologic stability of IL-9 or the IL-9 receptor.

In another embodiment, the agonists and antagonists of the invention are antibodies to IL-9 and the IL-9 receptor. The antibodies to IL-9 and its receptor may be either monoclonal or polyclonal made using standard techniques well known in the art (See Harlow & Lane's Antibodies - A Laboratory Manual (Cold Spring Harbor Laboratory, 1988). They can be used to block IL-9 from binding to the receptor. In one embodiment the antibodies interact with IL-9. In another embodiment the antibodies interact with the IL-9 receptor. The IL-9 used to elicit these antibodies can be any of the IL-9 varients discussed above.

Antibodies are also produced from peptide sequences of IL-9 or the IL-9 receptor using standard techniques in the art (see Protocols in Immunology, Chapt. 9, Wiley). The peptide sequences from the murine IL-9 receptor that can be used to produce blocking antisera have been identified as: GGQKAGAFTC (residues 1-10) (SEQ ID NO:19); LSNSIYRIDCHWSAPELGQESR (residues 11-32) (SEQ ID NO:20); and CESYEDKTEGEYYKSHWSEWS (residues 184-203 with a Cys residue added to the N-terminus for coupling the peptide to the carrier protein)(SEQ ID NO:21). In addition, an epitope that binds to a blocking antibody directed to the human IL-9 receptor has been identified as residues 8-14 of the mature human IL-9 receptor. (TCLTNNI)(SEQ ID NO:22) and two epitopes that bind to blocking antibodies directed to human IL-9 have also been' identified as residues 50-67 (CFSERLSQMTNTTMQTRY) (SEQ ID NO:23) and residues 99-116 (TAGNALTFLKSLLEIFQK) (SEQ ID NO:16) The human epitopes as well as the human peptides that correspond to the peptides that produce blocking antibodies in the murine sequences are most likely to be useful for the production of therapeutic antibodies.

In addition, the invention includes pharmaceutical compositions comprising the compounds of the invention together with a pharmaceutically acceptable carrier. Pharmaceutically acceptable carriers can be sterile liquids, such as water and oils, including those of petroleum, animal, vegetable or synthetic origin, such as peanut oil, soybean oil, mineral oil, sesame oil and the like. Water is a preferred carrier when the pharmaceutical composition is administered intravenously. Saline solutions and aqueous dextrose and glycerol solutions can also be employed as liquid carriers, particularly for injectionable solutions. Suitable pharmaceutical carriers are described in Martin, E.W., Remington's Pharmaceutical Sciences.

The antibodies used this invention may be administered systemically or topically, depending on such considerations as the condition to be treated, need for site-specific treatment, quantity of drug to be administered, and similar considerations.

Topical administration may be used. Any common topical formation such as a solution, suspension, gel, ointment, or salve and the like may be employed. Preparation of such topical formulations as are well described in the art of pharmaceutical formulations as exemplified, for example, by Remington's Pharmaceutical Science, Edition 17, Mack Publishing Company, Easton, Pa. For topical application, these compounds could also be administered as a powder or spray, particularly in aerosol form. The active ingredient may be administered in pharmaceutical compositions adapted for systemic administration. As is known, if a drug is to be a administered systemically, it may be confected as a powder, pill, tablets or the like, or as a syrup or elixir for oral administration. For intravenous, intraperitoneal or intra-lesional administration, the compound will be prepared as a solution or suspension capable of being administered by injection. In certain cases, it may be useful to formulate these compounds in suppository form or as an extended release formulation for deposit under the skin or intermuscular injection. In a preferred embodiment, the compounds of this invention be administered by inhalation. For inhalation therapy the compound may be in a solution useful for administration by metered dose inhalers, or in a form suitable for a dry powder inhaler.

An effective amount is that amount which will down regulate either the expression of IL-9 or the functions controlled by IL-9. A given effective amount will vary from condition to condition and in certain instances may vary with the severity of the condition being treated and the patient's susceptibility to treatment. Accordingly, a given effective amount will be best determined at the time and place through routine experimentation. However, it is anticipated that in the treatment of asthma-related disorders in accordance with the present invention, a formulation containing between 0.001 and 5 percent by weight, preferably about 0.01 to 1%, will usually constitute a therapeutically effective amount. When administered systemically, an amount between 0.01 and 100 mg per kg body weight per day, but preferably about 0.1 to 10 mg/kg, will effect a therapeutic result in most instances.

Applicant also provides for a method to screen for the compounds that down regulate the expression of IL-9 or the functions controlled by IL-9. One may determine whether the functions expressed by IL-9 are down-regulated using techniques standard in the art.⁵⁷⁻⁶⁰ In a specific embodiment, applicant provides for a method of identifying compounds with functions comparable to Met IL-9. Thus, in one embodiment, serum total IgE may be measured using techniques well known in the art⁴² to assess the efficacy of a compound in down regulating the functions of IL-9 in vivo. In another embodiment, bronchial hyperresponsiveness, bronchoalveolar lavage, and eosinophilia may be measured using techniques well known in the art⁴² to assess the efficacy of a compound in down regulating the functions of IL-9 in vivo. In yet another embodiment, the functions of IL-9 may be assessed in vitro. As is known to those in the art, human IL-9 specifically induces the rapid and transient tyrosine phosphorylation of multiple proteins in M07e cells. The tyrosine phosphorylation of Stat3 transcriptional factor appears to be specifically related to the actions of IL-9. Another method to characterize the function of IL-9 and IL-9-like molecules that depends on the "stable expression" of the IL-9 receptor uses the well known murine TS1 clones to assess human IL-9 function with a cellular proliferation assay.⁵⁹

The invention also includes a simple screening assay for saturable and specific ligand binding based on cell lines that express the IL-9 receptor.^{46,50} The IL-9 receptor is expressed in on a wide variety of cell types, including K562, C8166-45,B cells, T cells, mast cells, neutrophils, megakaryocytes (UT-7 cells),⁵³ the human megakaryoblastic leukemia cell lines MO7e⁵⁷, TF1,⁵⁹ macrophages, fetal thymocytes, the human kidney cell line 293,⁵³ and murine embryonic hippocampal progenitor cell lines.^{46,52,53} In another embodiment, soluble IL-9 receptor may be used to evaluate ligand binding and potential receptor antagonists.

The practice of the present invention will employ the conventional terms and techniques of molecular biology, pharmacology, immunology, and biochemistry that are within the ordinary skill of those in the art. See, for example, Sambrook et al., MOLECULAR CLONING: A LABORATORY MANUAL, 2d ed. Cold Spring Harbor Laboratory Press [1985], or Ausubel et al., CURRENT PROTOCOLS IN MOLECULAR BIOLOGY, John Wiley & Sons, Inc. [1994].

### EXAMPLE 1

### The role of IL-9 in murine models of asthma: The airway response of unsensitized animals

### Animals

Certified virus-free male mice ranging in age from 5 to 6 weeks were obtained from the Jackson Laboratory (Bar Harbor, ME). Animals were housed in high-efficiency particulate filtered air (HEPA) laminar flow hoods in a virus and antigen free facility and allowed free access to pelleted rodent chow and water for 3 to 7 days prior to experimental manipulation. The animal facilities were maintained at 22° C and the light:dark cycle was automatically controlled (10:14 h light:dark). Male and female DBA/2 (D2), C57BL/6 (B6), and (B6D2)F1 (F₁) mice 5 to 6 weeks of age were purchased from the Jackson Laboratory, Bar Harbor, ME, or the National Cancer Institute, Frederick, MD. BXD mice were purchased from the Jackson Laboratory, Bar Harbor, ME. Food and water were present ad libitum.

### Phenotyping and efficacy of pretreatment

To determine the bronchoconstrictor response, respiratory system pressure was measured at the trachea and recorded before and during exposure to the drug. Mice were anesthetized and instrumented as previously described. (Levitt RC, and Mitzner W, FASEB J 2:2605-2608 (1988); Levitt RC, and Mitzner W, J Appl Physiol 67(3): 1125-1132 (1989); Kleeberger S, Bassett D, Jakab GJ, and Levitt RC, Am J Physiol 258(2)L313-320 (1990); Levitt RC; Pharmacogenetics 1:94-97 (1991); Levitt RC, and Ewart SL; Am J of Respir Crit Care Med 151:1537-1542(1995); Ewart S, Levitt RC, and Mitzner W, In press, J Appl Phys (1995). Airway responsiveness was measured to one or more of the following: 5-hydroxytryptamine (5HT) (sigma). An additional branch construction that can be used is acetylcholine (sigma), atracurium (Glaxo welcome). A simple and repeatable measure of the change in Ppi following bronchoconstrictor challenge was used and which has been termed the Airway Pressure Time..Index (APTI) (Levitt RC, and Mitzner W, FASEB J 2:2605-2608 (1988); Levitt RC, and Mitzner W, J Appl Physiol 67(3): 1125-1132-(1989). The APTI was assessed by the change in peak inspiratory pressure (Ppi) integrated from the time of injection till the peak pressure returned to baseline or plateaued. The APTI was comparable to airway resistance (Rrs), however, the APTI includes an additional component related to the recovery from bronchoconstriction.

The strain distribution of bronchial responsiveness was identified in multiple inbred mouse strains in previous studies (Levitt RC, and Mitzner W, FASEB J 2:2605-2608 (1988); Levitt RC, and Mitzner W, J Appl Physiol 67(3): 1125-1132 (1989). The Rrs and/or APTI was determined in A/J, C3H/HeJ, DBA/2J, C57BL/6J mice.

Prior to sacrifice whole blood was collected for serum IgE measurements by needle puncture of the inferior vena cava in completely anesthetized animals. The samples were spun to separate cells and serum was collected and used to measure total IgE levels. Samples not measured immediately were frozen at -20°C.

Bronchoalveolar lavage and cellular analyses was preformed as described elsewhere (Kleeberger et al., 1990).

All IgE serum samples were measured using an ELISA antibody-sandwich assay. Microtiter plates (Corning #2585096,Corning, NY) were coated, 50 µl per well, with rat anti-mouse IgE antibody (Southern Biotechnology #1130-01, Birmingham, AL) at a concentration of 2.5 µg/ml in coating' buffer of sodium carbonate-sodium bicarbonate with sodium azide (Sigma #S-7795, #S-6014 and #S-8032, St Louis, MO). Plates were covered with plastic wrap and incubated at 4°C for 16 hours. The plates were washed three times with a wash buffer of 0.05% Tween-20 (Sigma #P-7949) in phosphate-buffered saline (BioFluids #313,Rockville, MD), incubating for five minutes for each wash. Blocking of nonspecific binding sites was accomplished by adding 200 µl per well 5% bovine serum albumin (Sigma #A-7888) in PBS, covering with plastic wrap and incubating for 2 hours at 37°C. After washing three times with wash buffer, duplicate 50 µl test samples were added to the wells. Test samples were assayed after being diluted 1:10, 1:50, and 1:100 with 5% BSA in wash buffer. In addition to the test samples a set of IgE standards (PharMingen #03121D, San Diego, CA) at concentrations from 0.8 ng/ml to 200 ng/ml in 5% BSA in wash buffer were assayed to generate a standard curve. A blank of no sample or standard was used to zero the plate reader (background). After adding samples and standards, the plate was covered with plastic wrap and incubated for 2 hours at room temperature. After washing three times with wash buffer, 50 ul of second antibody rat anti-mouse IgE-horseradish peroxidase conjugate (PharMingen #02137E) was added at a concentration of 250 ng/ml in 5% BSA in wash buffer. The plate was covered with plastic wrap and incubated 2 hours at room temperature. After washing three times with wash buffer, 100 ul of the substrate 0.5 mg/ml O-phenylaminediamine (Sigma #P-1526) in 0.1 M citrate buffer (Sigma #C-8532) was added to every well. After 5-10 minutes the reaction was stopped with 50 µl of 12.5% H₂SO₄ (VWR #3370-4, Bridgeport, NJ) and absorbance was measured at 490 nm on a Dynatech MR-5000 plate reader (Chantilly, VA). A standard curve was constructed from the standard IgE concentrations with antigen concentration on the x axis (log scale) and absorbance on the y- axis (linear scale). The concentration of IgE in the samples was interpolated from the standard curve.

### EXAMPLE 2

### The role of IL-9 in murine models of asthma: The airway response of sensitized animals

### Animals, phenotyping, and optimization of antigen sensitization

Animals and handling were essentially as described in Example 12. Sensitization with turkey egg albumin (OVA) and aerosol challenge was carried out to assess the effect on BHR, BAL, and serum IgE. OVA was injected I.P. (25 µg) day 0 prior to OVA or saline aerosolization. Mice were challenged with OVA or saline aerosolization which was given once daily for 5 to 7 days starting on either day 13 or 14. Phenotypic measurements of serum IgE, BAL, and BHR was carried out on day 21. The effect of a 7 day OVA aerosol exposure on bronchoconstrictor challenge with 5-HT and acetylcholine were evaluated along with serum total IgE, BAL total cell counts and differential cell counts, and bronchial responsiveness. The effect of antibody (Ab) or saline pretreatment on saline aerosol or OVA aerosol induced lung inflammation was examined by measuring BHR, BAL, and serum IgE. Ab were administered I.P. 2-3 days prior to aerosolization of saline or OVA.

Lung histology was carried out after the lungs are removed during deep anesthesia. Since prior instrumentation may introduce artifact, separate animals were used for these studies. Thus, a small group of animals was treated in parallel exactly the same as the cohort undergoing various pretreatments except these animals were not used for other tests aside from bronchial responsiveness testing. After bronchial responsiveness testing, the lungs were removed and submersed in liquid nitrogen. Cryosectioning and histologic examination were carried out in a routine fashion.

Polyclonal neutralizing antibodies for murine IL-9 were purchased from R & D systems, Minneapolis, MN and blocking antibodies for murine IL-9 receptor were produced for Magainin Pharmaceuticals Inc. by Lampine Biological Labatories, Ottsville, PA using peptide conjugates produced at Magainin. The polyclonal antisera were prepared in rabbits against peptide sequences from the murine IL-9 receptor. The peptides used to produce the antisera were: GGQKAGAFTC (residues 1-10)(SEQ ID NO:19); LSNSIYRIDCHWSAPELGQESR (residues 11-32) (SEQ ID NO:20); and CESYEDKTEGEYYKSHWSEWS (residues 184-203 with a Cys residue added to the N-terminus for coupling the peptide to the carrier protein)(SEQ ID NO:21). The antisera were generated using techniques described in Protocols in Immunology, Chapter 9, Wiley. Briefly, the peptides were coupled to the carrier protein, Keyhole Limpet, hemocyanin,(Sigma) through the side chain of the Cys residue using the bifunctional cross-linking agent MBS(Pierce). Peptide conjugates were used to immunize rabbits with appropriate adjuvents and useful antisera was obtained after several booster injections of the peptide conjugate. The antibodies were used therapeutically to down regulate the functions of IL-9 and assess the importance of this pathway to baseline lung responsiveness, serum IgE, and BAL in the unsensitized mouse. After Ab pretreatment on baseline BHR, BAL, and serum IgE levels relative to controls was determined. In additional experiments, recombinant human and murine IL-9 were administered I.P. 1 day before and daily during antigen sensitization (days 13-18). The animals were then phenotyped as described.

The phenotypic response of a representative animal treated with saline I.P. on day zero and challenged on days 14-20 with saline (as described in Example 12) is shown in Figure 20 panel 1 (top). Baseline (control) serum total IgE was 9.2 ng/ml. Bronchoalveolar lavage (BAL) total cell counts showed 182,500 cells per milliliter of BAL. These animals did not demonstrate bronchial hyperresponsiveness when compared to historical controls (Levitt RC, and Mitzner W, J Appl Physiol 67(3): 1125-1132;1989).

Figure 1 panel 2 (top middle) shows a representative animal from a group presensitized with OVA I.P on day zero and challenged with saline on days 14-20. These animals did not differ in their response to bronchoconstrictor, serum IgE, or BAL cell counts from the unsensitized mice (Figure 7 top panel).

Figure 1 panel 3 (bottom middle) shows a representative animal from those presensitized with OVA I.P on day zero and challenged with antigen (OVA) on days 14-20. These animals developed bronchial hyperresponsiveness (approximately two to three-fold over controls), elevated serum IgE (nearly one thousand-fold over controls), and increased numbers of inflammatory cells in the airway as demonstrated by elevated BAL cell counts (approximately thirty-fold) as compared to controls (Figure 20 top 2 panels). Most of the cells recruited to the airway as a result of this antigen challenge were eosinophils.

Figure 1 panel 4 (bottom) shows a representative animal from those presensitized with OVA I.P on day zero, pretreated with polyclonal neutralizing antibodies for murine IL-9 (approximately 200 µg/mouse I.P. in 0.5ml of PBS), and challenged with antigen (OVA) on days 14-20. These animals were protected from the response to antigen. They did not differ significantly in their bronchial responsiveness, serum IgE, or BAL cell counts from controls (Figure 1 top 2 panels).

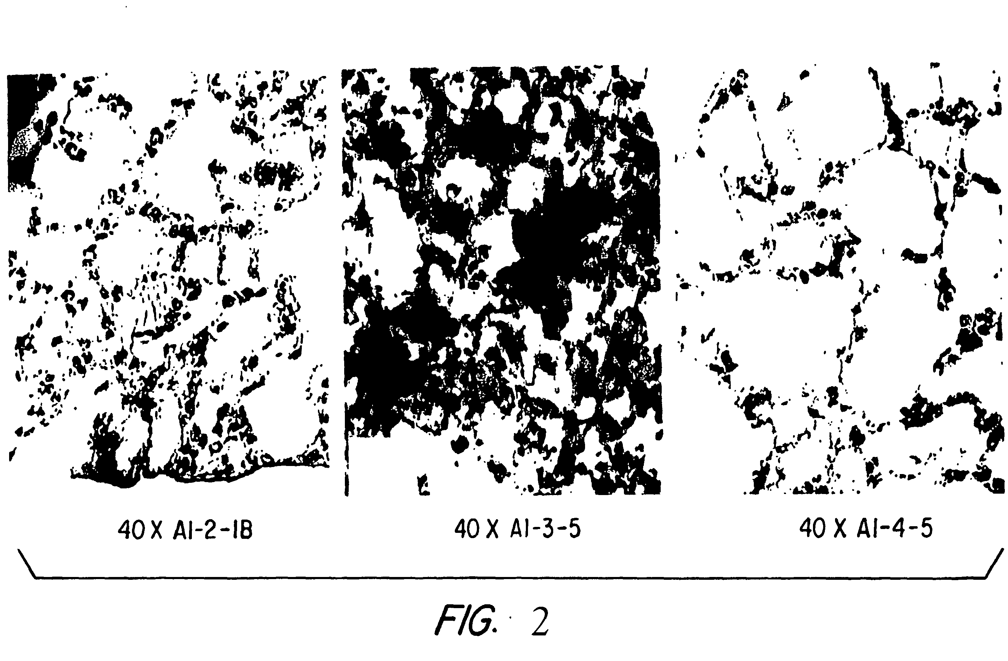

Figure 2 illustrates the effect of antigen challenge to OVA (as described above) with and without pretreatment with polyclonal neutralizing antibodies to murine IL-9 I.P. three days prior in representative animals. The left figure (A1-2-1B) is a histologic section from the lungs of control animals (sensitized to OVA but exposed only to a saline aerosol challenge). The middle figure (A1-3-5) is a histologic section from the lungs of animals sensitized to OVA and exposed to an OVA aerosol challenge. The right figure (A1-4-5) is a histologic section from the lungs of animals sensitized to OVA and exposed to an OVA aerosol challenge who were pretreated three days prior with polyclonal neutralizing antibodies to murine IL-9. Pretreatment with neutralizing antibody produced histological confirmation of complete protection from antigen challenge.

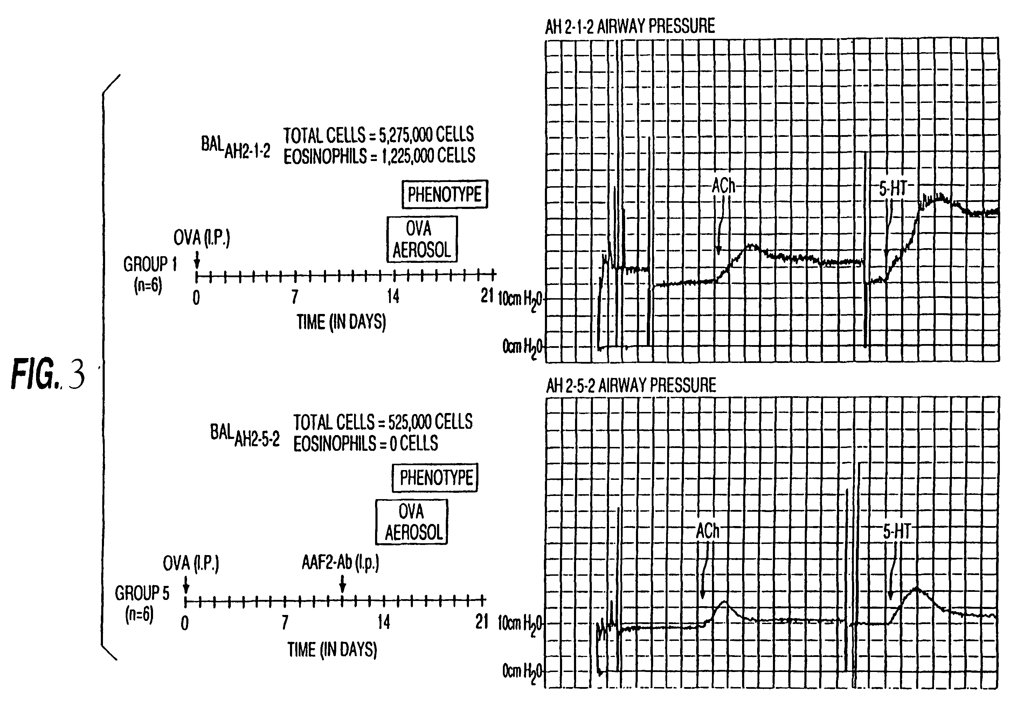

Figure 3 panel 1 (top) shows a representative animal from mice presensitized with OVA I.P on day zero and challenged with antigen (OVA) on days 13-18. These animals developed bronchial hyperresponsiveness (approximately two to three-fold over controls), and increased numbers of inflammatory cells including eosinphils in the airways as demonstrated by elevated BAL cell counts as compared to controls (Figure 20 top 2 panels). Many of the cells recruited to the airway as a result of this antigen challenge were eosinophils.

Figure 3 panel 2 (bottom) shows a representative animal from those presensitized with OVA I.P on day zero, pretreated with polyclonal neutralizing antibodies to the murine IL-9 receptor (approximately 1 mg/mouse I.P. in 0.5ml of PBS), and challenged with antigen (OVA) on days 13-18. This representative animal was protected from the response to antigen. This response did not differ significantly bronchial responsiveness, BAL cell counts from controls (Figure 1 top 2 panels). These data demonstrate the potential effectiveness of treating atopic allergy with antibodies to the IL-9 receptor.

### REFERENCES

1. Gergen PJ, and Weiss KB: The increasing problem of asthma in the United States. Am Rev Respir Dis 146:823-824, 1992.
2. Goodman and Gilman's The Pharmacologic Basis of Therapeutics, Seventh Edition, MacMillan Publishing Company, N.Y. USA, 1985.
3. Burrows B, Martinez FD, Halonen M, Barbee RA, and Cline MG: Association of asthma with serum IgE levels and skin-test reactivity to allergens. New Eng J Med 320:271-277, 1989.
4. Clifford RD, Pugsley A, Radford M, and Holgate ST: Symptoms, atopy, and bronchial response to methacholine in parents with asthma and their children. Arch Dis in Childhood 62:66-73, 1987.
5. Gergen PJ: The association of allergen skin test reactivity and respiratory disease among whites in the U.S. population. Arch Intern Med 151:487-492, 1991.
6. Burrows B, Sears MR, Flannery EM, Herbison GP, and Holdaway MD: Relationship of bronchial responsiveness assessed by methacholine to serum IgE, lung function, symptoms, and diagnoses in 11-year-old New Zealand children. J Allergy Clin Immunol 90:376-385, 1992.
7. Johannson SGO, Bennich HH, and Berg T: The clinical significance of IgE. Prog Clin Immunol 1:1-25, 1972.
8. Sears MR, Burrows B, Flannery EM, Herbison GP, Hewitt CJ, and Holdaway MD: Relation between airway responsiveness and serum IgE in children with asthma and in apparently normal children New Engl J Med 325(15):1067-1071, 1991.
9. Halonen M, Stern D, Taussig LM, Wright A, Ray CG, and Martinez FD: The predictive relationship between serum IgE levels at birtn and subsequent incidences of lower respiratory illnesses and eczema in infants. Am Rev Respir Dis 146:666-670, 1992.
10. Marsh DG, Meyers DA, and Bias WB: The epidemiology and genetics of atopic allergy. New Eng J Med 305:1551-1559, 1982.
11. Hopp RJ, Bewtra AK, Biven R, Nair NM, Townley RG. Bronchial reactivity pattern in nonasthmatic parents of asthmatics. Ann Allergy 1988;61:184-186.
12. Hopp RJ, Townley RG, Biven RE, Bewtra AK, Nair NM. The presence of airway reactivity before the development of asthma. Am Rev Respir Dis 1990;141:2-8.
13. Ackerman V, Marini M, Vittori E, et al. Detection of cytokines and their cell sources in bronchial biopsy specimens from asthmatic patients: relationship to atopic status, symptoms, and level of airway hyperresponsiveness. Chest 1994;105:687-696.
14. Hamid G, Azzawi M, Ying S, et al. Expression of mRNA for interleukin-5 in mucosal bronchial biopsies from asthma. J Clin Invest 1991;87:1541-1546.
15. Djukanovic R, Roche WR, Wilson JW, et al. Mucosal inflammation in asthma. Am Rev Respir Dis 1990;142:434-57.
16. Robinson DS, Hamid Q, Ying S, et al. Predominant TH2-like bronchoalveolar T lymphocyte population in atopic asthma. N Engl J Med 1992;326:298-304.
17. Robinson DS, Hamid Q, Ying S, et al. Prednisolone treatment in asthma is associated with modulation of bronchoalveolar lavage cell interleukin-4, interleukin-5, and interferon- cytokine gene expression. Am Rev Respir Dis 1993;148:401-406.
18. Robinson DS, Ying S, Bentley A, et al. Relationship among numbers of bronchoalveolar lavage cells expressing messenger ribonucleic acid for cytokines, asthma symptoms, and airway methacholine responsiveness in atopic asthma. J Allergy Clin Immunol 1993;92:397-403.
19. Sears M, Burrows B, Flannery EM, Herbison GP, Hewitt CJ, Holdaway MD. Relation between airway responsiveness and serum IgE in children with asthma and in apparently normal children. N Engl J Med 1991;325:1067-1071.
20. Burrows B, Sears MR, Flannery EM, Herbison GP, Holdaway MD. Relationship of bronchial responsiveness assessed by methacholine to serum IgE, lung function, symptoms, and diagnoses in 11-year-old New Zealand children. J Allergy Clin Immunol 1992;90:376-385.
21. Clifford RD, Pugsley A, Radford M, Holgate ST. Symptoms, atopy, and bronchial response to methacholine in parents with asthma and their children. Arch Dis Childhood 1987;62:66-73.
22. O'Connor GT, Sparrow D, and Weiss ST: The role of allergy and nonspecific BHR in the pathogenesis of COPD. Am Rev Respir Dis 140:225-252, 1989.
23. Cogswell JJ, Halliday DF, and Alexander JR: Respiratory infections in the first year of life in children at the risk of developing atopy. Brit Med J 284:1011-1013, 1982.
24. Boushey HA, Holtzman MJ, Sheller JR, and Nadel JA: BHR. Am Rev Respir Dis 121:389-413, 1980.
25. Cookson WOCM, and Hopkin JM: Linkage between immunoglobin E responses underlying asthma and rhinitis and chromosome 11q. Lancet 1292-1295,1989.
26. Moffatt MF, Sharp PA, Faux JA, Young RP, Cookson WOCM, and Hopkins JM; Factors confounding genetic linkage between atopy and chromosome 11q. Clin Exp Allergy 22:1046-1051, 1992.
27. Amelung P, Panhuysen C, Postma DS, Levitt RC, Koeter GH, Francomano C, Bleeker ER, and Meyers DA: Atopy, asthma and bronchial hyperresponsiveness: Exclusion of linkage to markers on chromosome 11q and 6p. Clin Exper Allergy 22:1077-1084, 1992.
28. Rich SS, Roitman-Johnson B, Greenberg B, Roberts S, and Blumenthal MN: Genetic evidence of atopy in three large kindreds: no evidence of linkage to D11S97. Clin Exp Allergy 22:1070-1076, 1992.
29. Lympany P, Welsh K, MacCochrane G, Kemeny DM, and Lee TH: Genetic analysis using DNA polymorphism of the linkage between chromosome 11q13 and atopy and BHR to methacholine. J Allergy Clin Immunol 89:619-628, 1992a.
30. Lympany P, Welsh KI, Cochrane GM, Kemeny DM, and Lee TH: Genetic analysis of the linkage between chromosome 11q and atopy. Clin Exp Allergy 22:1085-1092, 1992b.
31. Hizawa N, Yamagushi E, Ohe M, Itoh A, Furuya K, Ohnuma N, and Kawakami Y: Lack of linkage between atopy and locus 11q13. Clinical and Experimental Allergy 22:1065-1069, 1992.
32. Sanford AJ, Shirakawa T, Moffatt MF, Daniels SE, Ra C, Faux JA, Young RP, Nakamura Y, Lathrop GM, Cookson WOCM, and Hopkin JM: Localization of atopy and b subunit of high-affinity IgE receptor (FceR1) on chromosome 11q. Lancet 341:332-334, 1993.
33. Shirakawa T, Airong L, Dubowitz M, Dekker JW, Shaw AE, Faux JA, Ra C, Cookson WOCM, and Hopkin JM: Association between atopy and variants of the β subunit of the high-affinity immunoglobulin E receptor. Nature Genetics 7:125-130, 1994.
34. Marsh DG, Bias WB, and Ishizaka K: Genetic control of basal serum immunoglobulin E level and its effect on specific reaginic sensitivity. Proc Natl Acad Sci USA 71:3588-3592, 1974.
35. Gerrard JW, Rao DC, and Morton NE: A genetic study of IgE. Am J Hum Genet 30:46-58, 1978.
36. Meyers DA, Beaty TH, Freidhoff LR, and Marsh DG: Inheritance of serum IgE (basal levels) in man. Am J Hum Genet 41:51-62, 1987.
37. Meyers DA, Bias WB, and Marsh DG: A genetic study of total IgE levels in the Amish. Hum Hered 32:15-23, 1982.
38. Martinez FD, Holberg CJ, Halonen M, Morgan WJ, Wright AL, and Taussig LM: Evidence for mendelian inheritance of serum IgE levels in Hispanic and Non-hispanic white families. Am J Hum Genet 55:555-565, 1994.
39. Blumenthal MN, Namboordiri KK, Mendell N, Gleich G, Elston RC, and Yunis E: Genetic transmission of serum IgE levels. Am J Med Genet 10:219-228, 1981.
40. The Genome Data Base. The Welch Library, The Johns Hopkins Medical Institutions, Baltimore, Maryland, USA.
41. Marsh DG, Neely JD, Breazeale DR, et al. Linkage analysis of IL4 and other chromosome 5q31.1 markers and total serum immunoglobulin E concentrations. Science 1994;264:1152-1156.
42. Meyers DA, Postma DS, Panhuysen CIM, et al. Evidence for a locus regulating total serum IgE levels mapping to chromosome 5. Genomics 1994;23:464 470.41.
43. Doull, I., Lawrence, S., Watson, M., Begishvili, T., Beasley, R., Lampe, F., Holgate, S.T., Morton, N.E. Allelic association of makers on chromosome 5q and 11q with atopy and bronchial hyperresponsiveness. Am J Respir Crit Care Med, 1996:153:1280-1284.
44. Ott J. Analysis of human genetic linkage. Baltimore, Maryland: The Johns Hopkins University Press, 1991.
45. Renauld, J-C, Houssiau, F, Druez, C. Interleukin-9. Int Rev Exp Pathology 1993:39A: 99-109.
46. Renauld, J-C, Kermouni, A, Vink, A, Louahed, J, Van Snick, J. Interleukin-9 and its receptor: involvement in mast cell differentiation and T cell oncogenesis. J Leukoc Biol 1995;57:353-360.
47. Hultner, L, Moeller, J, Schmitt, E, Jager, G, Reisbach, G, Ring, J. Dormer, P. Thiol-sensitive mast cell lines derived from mouse bone marrow respond to a mast cell growth-enhancing activity different from both IL-3 and IL-4. J Immunol 1989;142:3440-3446.
48. Dugas, B, Renauld, J-C, Pene, J, Bonnefoy, J, Peti-Frere, C, Braquet, P, Bousquet, J, Van Snick, J, Mencia-Huerta, JM. Interleukin-9 potentiates the interleukin-4-induced immunoglobulin [IgG, IgM and IgE] production by normal human B lymphocytes. Eur J Immunol 1993;23:1687-1692.
49. Petit-Frere, C, Dugas, B, Braquet, P, Mencia-Huerta, JM. Interleukin-9 potentiates the interleukin-4-induced IgE and IgG1 release from murine B lymphocytes. Immunology 1993;79:146-151.
50. Behnke, JM, Wahid, FN, Grencis, RK, Else, KJ, Ben-Smith, AW, Goyal, PK. Immunological relationships during primary infection with Heligmosomoides polygyrus [Nematospiroides dubius]: downregulation of specific cytokine secretion [IL-9 and IL-10] correlates with poor mastocytosis and chronic survival of adult worms. Parasite Immunol 1993;15:415-421.
51. Gessner, A, Blum, H, Rollinghoff, M. Differential regulation of IL-9 expression after infection with Leischmania major in susceptible and resistant mice. Immunobiology 1993;189:419-435.
52. Renauld J-C, Druez C, Kermouni A, et al. Expression cloning of the murine and human interleukin 9 receptor cDNAs. Proc Natl Acad Sci 89:5690-5699(1992).
53. Chang M-S, Engel G, Benedict C et al. Isolation and characterization of the Human interleukin-9 receptor gene. Blood 83:3199-3205(1994).
54. Renauld J-C, Goethals A, Houssiau F, et al. Human P40/IL-9. Expression in activated CD4+ T cells, Genomic Organization, and Comparison with the Mouse Gene. J Immunol 144:4235-4241(1990).
55. Kelleher K, Bean K, Clark SC, et al. Human interleukin-9: genomic sequence, chromosomal location, and sequences essential for its expression in human T-cell leukemia virus (HTLV-I-transformed human T cells. Blood 77:1436-1441(1991).
56. Houssiau FA, Schandene L, Stevens M, et al. A cascade of cytokines is responsible for IL-9 expression in human T cells. Involvement of IL-2, IL-4, and IL-10. J of Immunol. 154:2624-2630(1995).
57. Miyazawa K, Hendrie PC, Kim Y-J, et al. Recombinant human interleukin-9 induces protein tyrosine phosphorylation and synergizes with steel factor to stimulate proliferation of the human factor-dependent cell line, M07e. Blood 80:1685-1692(1992).
58. Yin T, Tsang M L-S, Yang Y-C. JAK1 kinase forms complexes with interleukin-4 receptor and 4PS/insulin receptor substrate-1-like protein and is activated by interleukin-4 and interleukin-9 in T lymphocytes. J Biol Chem 269:26614-26617(1999).
59. Renauld J-C, Druez C, Kermouni A, et al. Expression cloning of the murine and human interleukin 9 receptor cDNAs. Proc Natl Acad Sci 89:5690-5699(1992).
60. Chang M-S, Engel G, Benedict C et al. Isolation and characterization of the Human interleukin-9 receptor gene. Blood 83:3199-3205(1994).
61. Kreitman RJ, Puri RK, Leland P, et al. Site-specific conjugation to interleukin4 containing mutated cysteine residues produces interleukin 4-toxin conjugates with improved binding and activity. Biochemistry 33:11637-11644(1994)
62. Simoncsits A, Bristulf J, Tjornhammar ML, et al. Deletion mutants of human interleukin 1 beta significantly reduced agonist properties: search for the agonist/antagonist switch in ligands to the interleukin 1 receptors. Cytokine 6:206-214(1994).
63. Zav'yalov VP, Navolotskaya EV, Isaev IS, et al. Nonapeptide corresponding to the sequence 27-35 of the mature human IL-2 efficiently competes with rIL-2 for binding to thymocyte receptors. Immunol Lett 31:285-288(1992).
64. Chu JW, and Sharom FJ. Glycophorin A interacts with interleukin-2 and inhibits interleukin-2-dependent T-lymphocyte proliferation. Cell Immunol 145:223-239(1992).
65. Alexander AG, Barnes NC, Kay AB. Trial of cyclosporin in corticosteroid-dependent chronic severe asthma. Lancet 339:329-328(1992).
66. Morely J. Cyclosporin A in asthma therapy: a pharmacological rationale. J Autoimmun 5 Suppl A:265-269(1992).
67. Lander, E.S., Botstein, D. Mapping Mendelian factors underlying quantitative traits using RFLP linkage maps. Genetics 121, 185-199(1989).
68. Soller , M., Brody, T. On the power of experimental designs for the detection of linkage between maker loci and quantitative loci in crosses between inbred lines. Theor Appl Genet 47:35-39(1976).
69. Kvaloy K, Galvagni F, and Brown WRA. The sequence organization of the long arm pseudoautosomal region of the human sex chromosomes. Hum Mol Genet 3:771-778(1999).
70. Freije D, Helms C, Watson MS, et al. Science 258:1789-1787(1992).
71. Weber JL, May PE. Abundant class of human DNA polymorphisms which can be typed using the polymerase chain reaction. Am J Human Genet 1989;44:388-396.
72. Saiki KK, Gelfand DH, Stoffel S, et al. Primer-directed enzymatic amplification of DNA with a thermostable DNA polymerase. Science 1988;239:487-491.
73. Sheffield VC, Beck JS, Kwitek AE, Sandstrom DW, and Stone EM: The sensitivity of single-strand conformation polymorphism analysis for the detection of single base substitutions. Genomics 16:325-332, 1993.
74. Orita M, Suzuki Y, Sekiya T, and Hayashi K: Rapid and sensitive detection of point mutations and DNA polymorphisms using the polymerase chain reaction. Genomics 5:874-9, 1989.
75. Sarkar G, Yoon H-S, and Sommer SS: Dideoxy fingeringprint (ddF): A rapid and efficient screen for the presence of mutations. Genomics 13:441-443, 1992.
76. Cotton RG: Detection of single base changes in nucleic acids. Biochemical Journal 263(1):1-10, 1989.
77. Schwengel D, Nouri N, Meyers D, and Levitt RC: Linkage mapping of the human thromboxane A2 receptor (TBXA2R) to chromosome 19p13.3 using transcribed 3' untranslated DNA sequence polymorphisms. Genomics 18:212-215, 1993.
78. SAGE, Statistical Analysis for Genetic Epidemiology (1992). Release 2.1. Computer program package available from the Department of Biometry and Genetics, LSU Medical Center, New Orleans, LA.
79. Postma DS, Bleecker ER, Holroyd KJ, Amelung PJ, Panhuysen CIM, Xu J, Meyers DA, Levitt RC: Genetic Susceptibility to Asthma: Bronchial Hyperresponsiveness Coinherited with a Major Gene for Atopy. N Engl J Med 333:894-900 (1995).
80. Xu J, Levitt RC, Panhuysen CIM, Postma DS, Taylor EW, Amelung PJ, Holroyd KJ, Bleecker ER, Meyers DA: Evidence for two-unlinked loci regulating serum total IgE levels. Am J Hum Genet 57:425-430 (1995).
81. Meyers DA, Xu J, Holroyd KJ, Panhuysen CIM, Amelung PJ, Postma DS, Levitt RC, Bleecker ER. Two locus segregation and linkage analysis for total serum IgE levels. Clin Exp Allergy 25:113-115 (1995).
82. Bleecker ER, Amelung PJ, Levitt RC, Postma DS, Meyers DA. Evidence for linkage of total serum IgE and bronchial hyperresponsiveness to chromosome 5q: a major regulatory locus important in asthma. Clin Exp Allergy 25:84-88 (1995).
83. Panhuysen CIM, Levitt RC, Postma DS, et al., Evidence for a susceptibility locus for asthma mapping to chromosome 5q. Journal of Investigative Medicine 43:281A (1995).
84. Levitt RC, Eleff SM, Zhang L-Y, Kleeberger SR, and Ewart SL. Linkage homology for bronchial hyperresponsiveness between DNA markers on human chromosome 5q31-q33 and mouse chromosome 13. Clin Exp Allergy 25:61-63 (1995).
85. Yang et al., U.S. Patent No. 5,414,071 Human cytokine IL-9 (May 9, 1995).
86. Alms W and White B. Human interleukin variants generated by alternative splicing PCT/US95/04094 (WO 95/27052).
87. Cytokine handbook, Angus Tnomson (1994).
88. Martinati LC, Trabetti E, Casartelli A, Boner AL, Pignatti PF. Affected sib-pair and mutation analyses of the high affinity IgE receptor beta chain locus in Italian families with atopic asthmatic children. Am J Respir Crit Care Med, 1996;153:1682-1685.
89. Kauvar, M. Lawrence, Peptide mimetic drugs: A comment on progress and prospects, Nature Biotechnology Volume 14, June 1996.

Other embodiments of the invention described above and will be apparent to those skilled in the art from consideration of the specification and practice of the invention disclosed within. It is intended that the specification and examples considered as exemplary only, with true scope and spirit of the invention being indicated by the following claims:

## Claims

1. Use of an antibody, which is an anti-interleukin-9 antibody or an anti-interleukin-9 receptor antibody, that blocks interleukin-9 binding to the interleukin-9 receptor, for the preparation of a medicament for the treatment of asthma.

2. The use according to claim 1, wherein said antibody is in an amount sufficient to down regulate the activity of interleukin-9.

3. The use according to any of claims 1 or 2, wherein said antibody reduces eosinophilia in the bronchial lavage in a patient.

4. The use according to claim 3, wherein said antibody is a monoclonal antibody.

5. The use according to claims 1 to 3, wherein said antibody is an antigen binding fragment.

6. The use according to any of claims 1 to 5, wherein said medicament is administered by injection.

7. The use according to claim 6, wherein said medicament is administered by intravenous injection.

8. The use according to claim 6, wherein said medicament is administered by subcutaneous injection.

9. The use according to claims 1 to 5, wherein said medicament is administered by inhalation.

10. The use according to claim 9, wherein said medicament is administered by an inhalation device.

11. The use according to claim 10, wherein said medicament is administered by a metered dose inhaler.

12. The use according to claim 10, wherein said medicament is administered by a dry powder inhaler.

## Patentansprüche

1. Verwendung eines Antikörpers, wobei es sich um einen Anti-Interleukin-9-Antikörper oder einen Anti-Interleukin-9-Rezeptor-Antikörper handelt, der die Interleukin-9-Bindung an den Interleukin-9-Rezeptor blockiert, zur Herstellung eines Medikaments für die Behandlung von Asthma.

2. Verwendung gemäss Anspruch 1, wobei der Antikörper in einer ausreichenden Menge vorliegt, um die Aktivität von Interleukin-9 herabzuregulieren.

3. Verwendung gemäss einem der Ansprüche 1 oder 2, wobei der Antikörper die Eosinophilen in einer Bronchiallavage bei einem Patienten reduziert.

4. Verwendung gemäss Anspruch 3, wobei der Antikörper ein monoklonaler Antikörper ist.

5. Verwendung gemäss einem der Ansprüche 1 bis 3, wobei der Antikörper ein Antigen-Bindungsfragment ist.

6. Verwendung gemäss einem der Ansprüche 1 bis 5, wobei das Medikament durch Injektion verabreicht wird.

7. Verwendung gemäss Anspruch 6, wobei das Medikament durch intravenöse Injektion verabreicht wird.

8. Verwendung gemäss Anspruch 6, wobei das Medikament durch subkutane Injektion verabreicht wird.

9. Verwendung gemäss einem der Ansprüche 1 bis 5, wobei das Medikament durch Inhalation verabreicht wird.

10. Verwendung gemäss Anspruch 9, wobei das Medikament durch eine Inhalationsvorrichtung verabreicht wird.

11. Verwendung gemäss Anspruch 10, wobei das Medikament durch eine Dosierdosisinhalationsvorrichtung verabreicht wird.

12. Verwendung gemäss Anspruch 10, wobei das Medikament durch eine Trockenpulverinhalationsvorrichtung verabreicht wird.

## Revendications

1. Utilisation d'un anticorps qui est un anticorps anti-interleukine-9 ou un anticorps du récepteur de anti-interleukine-9, qui bloque la fixation de l'interleukine-9 au récepteur de l'interleukine-9, pour la préparation d'un médicament destiné au traitement de l'asthme.

2. Utilisation selon la revendication 1, dans laquelle ledit anticorps est en quantité suffisante pour réguler négativement l'activité de l'interleukine-9.

3. Utilisation selon l'une quelconque des revendications 1 et 2, dans laquelle ledit anticorps réduit l'éosinophilie dans le lavage bronchique chez un patient.

4. Utilisation selon la revendication 3, dans laquelle ledit anticorps est un anticorps monoclonal.

5. Utilisation selon l'une quelconque des revendications 1 à 3, dans laquelle ledit anticorps est un fragment de liaison à l'antigène.

6. Utilisation selon l'une quelconque des revendications 1 à 5, dans laquelle ledit médicament est administré par injection.

7. Utilisation selon la revendication 6, dans laquelle ledit médicament est administré par injection intraveineuse.

8. Utilisation selon la revendication 6, dans laquelle ledit médicament est administré par injection sous-cutanée.

9. Utilisation selon l'une quelconque des revendications 1 à 5, dans laquelle ledit médicament est administré par inhalation.

10. Utilisation selon la revendication 9, dans laquelle ledit médicament est administré par un dispositif d'inhalation.

11. Utilisation selon la revendication 10, dans laquelle ledit médicament est administré par un inhalateur-doseur.

12. Utilisation selon la revendication 10, dans laquelle ledit médicament est administré par un inhalateur de poudre sèche.
